# EUROPÄISCHE PATENTSCHRIFT

(11) **EP 0 699 653 B1**
(45) Veröffentlichungstag und Bekanntmachung des Hinweises auf die Patenterteilung: **07.01.1998**
(21) Anmeldenummer: 95113737.1
(22) Anmeldetag: 01.09.1995
(51) Int. Cl.: C07C 209/60, C07C 211/07, C07C 211/35

(54) **Verfahren zur Herstellung von Aminen**
Method for the preparation of amines
Procédé pour la préparation des amines

(30) Priorität: 01.09.1994 DE 4431093
(43) Veröffentlichungstag der Anmeldung: 06.03.1996
(73) Patentinhaber: BASF Aktiengesellschaft, 67063 Ludwigshafen (DE)
(72) Erfinder: Dingerdissen, Uwe, Dr., D-64342 Seeheim-Jugenheim (DE); Lauth, Günter, Prof. Dr., D-23562 Lübeck (DE); Henne, Andreas, Dr., D-67433 Neustadt (DE); Stops, Peter, D-67122 Altrip (DE); Eller, Karsten, Dr., D-67059 Ludwigshafen (DE); Gehrer, Eugen, Dr., D-67069 Ludwigshafen (DE)

(56) Entgegenhaltungen:
- EP-A- 0 039 918
- EP-A- 0 132 736
- EP-A- 0 133 938
- WO-A-93/17995
- DE-C- 3 634 247

## Beschreibung

Die vorliegende Erfindung betrifft ein Verfahren zur Herstellung von Aminen durch Umsetzung von Olefinen mit Ammoniak, primären oder sekundären Aminen bei erhöhten Temperaturen und Drücken in Gegenwart von mesoporösen und gegebenenfalls mikroporösen, röntgenamorphen Katalysatoren.

Die Addition von Ammoniak oder analog reagierenden Aminen an Olefine in Gegenwart von zeolithischen Katalysatoren, insbesondere Bor-, Alumo- und Eisensilikatzeolithe vom Pentasiltyp als aktive und langzeitstabile Katalysatoren ist aus der DE-A-33 26 579, DE-A-33 27 000 und DE-A-36 34 247 bekannt.

Nachteilig an der Zeolith-Katalyse ist die relativ aufwendige Herstellung der Pentasil-Zeolithe durch hydrothermale Kristallisation. Die selektive Synthese eines Molekularsiebs erfordert die genaue Einhaltung vieler Parameter, wie etwa der Kristallisationszeit, der Kristallisationstemperatur oder der Alterungsschritte.

Die bei einer Zeolithsynthese üblicherweise eingesetzten strukturdirigierenden Verbindungen (Template) müssen nach der Kristallisation entfernt werden. Die Entfernung des Templats geschieht in der Regel durch Kalzinierung, wobei die organische Verbindung oxidativ abgebaut wird.

Zeolithe besitzen eine sehr enge Porengrößenverteilung. Die Porengrößen variieren je nach Zeolithtyp von 4 bis ca. 12 Å.

Bei einer Zeolith-katalysierten Reaktion haben nur solche Moleküle Zugang zu den katalytisch aktiven Zentren im Inneren des Zeoliths, welche kleiner sind als die Porendimensionen. Reaktanden mit größeren Abmessungen sind vom Inneren der Poren ausgeschlossen.

Der vorliegenden Erfindung lag daher die Aufgabe zugrunde, den zuvor genannten Nachteilen abzuhelfen.

Demgemäß wurde ein neues und verbessertes Verfahren zur Herstellung von Aminen der allgemeinen Formel I 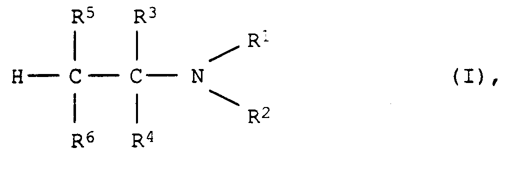 in der
- R¹,R²,R³,R⁴,R⁵,R⁶: Wasserstoff, C₁- bis C₂₀-Alkyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl, C₇- bis C₂₀-Aralkyl und heterocyclische Reste
bedeuten, gefunden, welches dadurch gekennzeichnet ist, daß man Olefine der allgemeinen Formel II 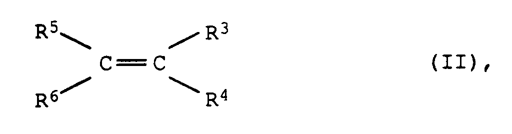 in der R³, R⁴, R⁵ und R⁶ oben genannten Bedeutung haben, mit Ammoniak, primären oder sekundären Aminen der allgemeinen Formel III 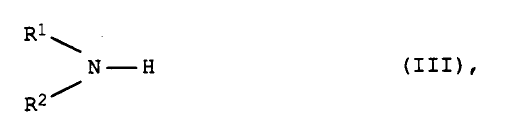 in der R¹ und R² die oben genannten Bedeutungen hat, bei Temperaturen von 200 bis 400°C und Drücken von 1 bis 700 bar in Gegenwart von mesoporösen und gegebenenfalls mikroporösen, röntgenamorphen Katalysatoren der Zusammensetzung

a MO₂ * b Q₂O₃ * c P₂O₅ (IV),

in der Q für Aluminium, Bor, Chrom, Eisen oder Gallium und M für Silicium, Titan oder Germanium steht, und das Molverhältnis a zu b 0,5:1 bis 1000:1 und das von c zu b 0 bis 2:1 beträgt, mit einer spezifische BET-Oberfläche von 200 bis 1000 m²/g, umsetzt.

Das erfindungsgemäße Verfahren zur Herstellung von Aminen I kann wie folgt durchgeführt werden:

Die Umsetzung erfolgt durch Kontakt einer Mischung aus einem Olefin II und einem Amin III an den beschriebenen meso- und gegebenenfalls mikroporösen röntgenamorphen Katalysatoren bei Temperaturen von 200 bis 400°C und Drücken von 0,01 bis 700 bar.

Die Reaktion kann sowohl in der Flüssigphase (Suspensions-, Riesel- oder Sumpffahrweise) bei Temperaturen von 200 bis 400°C und Drücken von 1 bis 700 bar diskontinuierlich, bevorzugt kontinuierlich durchgeführt werden. Die Katalysatorbelastung sollte in aller Regel bei WHSV = 0,1 bis 20 h⁻¹, bevorzugt 0,5 bis 5 h⁻¹ (g Einsatzgemisch/g Katalysator und Stunde) liegen.

Nach der Umsetzung werden die entstandenen Produkte durch übliche Techniken z. B. durch Destillation aus dem Reaktionsgemisch isoliert; nichtumgesetztes Einsatzgemisch wird gegebenenfalls in die erfindungsgemäße Umsetzung zurückgeführt.

In einer besonders bevorzugte Ausführungsform werden die gasförmigen Reaktionsprodukte direkt (sofort) nach Verlassen des Reaktors in eine Trennung eingebracht und anschließend in ihre Einzelkomponenten zerlegt werden. Eine derartige Trennung kann z.B. in einer Fraktionierkolonne durchgeführt werden. Dies ist empfehlenswert, um eine Rückreaktion zu unterbinden und um einen hohen Umsatz zu erzielen.

Als Katalysatoren für das erfindungsgemäße Verfahren eignen sich amorphe Katalysatoren einer chemischen Zusammensetzung der Formel IV, welche aus einem nach der Sol-Gel-Methode hergestellten amorphen Gel hergestellt wurden, sehr gut für die Aminierungsreaktion.

Katalysatoren dieser Art besitzen eine hohe Oberfläche, sowie eine hohe Konzentration an aciden Zentren; sie sind im Vergleich zu Zeolithen relativ einfach herzustellen, insbesondere benötigt ihre Herstellung keinen hydrothermalen Behandlungsschritt.

Die erfindungsgemäßen Katalysatoren sind mikro- und/oder mesoporös; daher können auch voluminösere Reaktanden in das Porensystem eindringen und reagieren.

### Herstellung der erfindungsgemäßen Gele

Die erfindungsgemäßen Gele lassen sich durch Hydrolyse einer Synthesemischung, welche neben einem Lösungsmittel mindestens folgende Komponenten enthält:
- eine lösliche, hydrolysierbare Q-Verbindung, und
- eine lösliche, hydrolysierbare M-Verbindung
wobei
Q für mindestens ein dreiwertiges Element, ausgewählt aus der Gruppe Aluminium, Bor, Chrom, Eisen, Gallium steht, M für mindestens ein vierwertiges Element, ausgewählt aus der Gruppe Silicium, Titan, Germanium steht, herstellen.

Besonders geeignete Katalysatoren sind solche der Zusammensetzung a SiO₂ * b Al₂O₃, wobei das Molverhältnis a zu b = 0,5:1 bis 1000:1 beträgt, solche der Zusammensetzung a SiO₂ * b B₂O₃, wobei das Molverhältnis a zu b = 0,5:1 bis 1000:1 beträgt, solche der Zusammensetzung a SiO₂ * b Al₂O₃ * c P₂O₅, wobei das Molverhältnis a zu b = 10:1 bis 1000:1 und c zu b = 0,5:1 bis 2:1 beträgt.

Daneben kann die Synthesemischung noch weitere Komponenten, wie z.B. Katalysatoren, weitere Lösungsmittel und/oder porenregulierende Verbindungen, enthalten. Insbesondere kann ein hydrolysebeschleunigender Katalysator - wie z.B. anorganische oder organische Säuren und Basen z.B. Alkalihydroxide wie Kaliumhydroxid oder Natriumhydroxid; Ammoniak; Mineralsäuren wie Salzsäure oder Flußsäure; organische Säuren wie Essigsäure; organische Basen wie Morpholin, Amine oder Tetraalkylammoniumhydroxide - anwesend sein.

Der durch die zugesetzten Säuren oder Basen veränderte pH-Wert der Synthesemischung kann auch die Porenstruktur des Gels bzw. des späteren Katalysators wesentlich beeinflussen.

Neben dem zur Hydrolyse notwendigen Wasser können weitere Lösungsmittel wie etwa Alkohole, Ether oder Ketone anwesend sein.

Vorteilhaft kann ferner die Anwesenheit von porenregulierenden Verbindungen sein. Derartige Verbindungen sind heteroatomhaltige organische Verbindungen, wie z.B. primäre, sekundäre oder tertiäre Amine bzw. deren Ammoniumverbindungen, Alkohole oder Polyalkohole, Ether oder Polyether, Verbindungen mit Carbonyl- oder Carboxylgruppen, Phosphoniumverbindungen.

Diese Verbindungen beeinflussen die Ausbildung der Porenstruktur des Gels; ihre Wirkung ist für Alumosilikat-Gele z.B. in Appl. Catal. 12 (1984) 327 bis 357 beschrieben.

Manchmal werden diese porenregulierenden Verbindungen auch als "Template" bezeichnet. In einer besonderen Anwendungsform werden diese Template in einem ersten Schritt zunächst mit den löslichen Q- und/oder M-Verbindungen zur Reaktion gebracht und erst in einem zweiten Schritt zu einem Gel verarbeitet [Chemtech (1993) 26 bis 31].

Die porenregulierende Verbindung kann gleichzeitig als Katalysator für den Hydrolyseprozeß, als pH-regulierendes Mittel und/oder als Lösungsmittel dienen.

Die lösliche hydrolysierbare M-Verbindung kann eine Germanium-, Titan- oder Siliciumverbindung sein, welche sich in einem Lösungsmittel zumindest kolloidal löst und nach der Hydrolyse das entsprechende Oxid ergibt. Die lösliche hydrolysierbare Verbindung kann anorganischer oder organischer Natur sein. Bevorzugt kommen als M-Verbindungen Siliciumverbindungen, wie Silikate (z.B. Natriumsilikat), Kieselsäureester (z.B. Tetraethylorthosilikat) und Kieselsol in Frage. Die lösliche hydrolysierbare Q-Verbindung kann eine Aluminium-, Bor-, Chrom-, Eisen- oder Galliumverbindung sein, welche sich in einem Lösungsmittel zumindest kolloidal löst und nach der Hydrolyse das entsprechenden Oxid ergibt. Die löslichen hydrolysierbaren Verbindungen können anorganischer oder organischer Natur sein. Bevorzugt können als Q-Verbindungen Aluminium- und/oder Borverbindung Verwendung finden. Als Aluminiumverbindungen kommen z.B. Aluminate (z.B. Natriumaluminat), Aluminiumsalze (z.B. Aluminiumsulfat), Aluminiumhydroxid, Aluminiumalkoxide (z.B. Aluminiumtriisopropanolat) in Frage.

Als Borverbindungen können z.B. Borsäure oder deren Ester eingesetzt werden.

Soll das Gel Phosphor enthalten, so können als lösliche Phosphorquelle z.B. Phosphorsäuren oder lösliche Phosphate eingesetzt werden.

Zusätzlich kann die Synthesemischung auch weitere Metalle in gelöster Form enthalten.

### Herstellung der Gele

Die Synthesemischung enthält die oben beschriebenen Bestandteile in Konzentrationen, welche einer oxidischen Zusammensetzung wie in Formel IV entsprechen. Diese Mischung wird in der Regel nach einer Zeit von wenigen Minuten bis mehreren Tagen in ein Gel übergehen. Die Gelbildung kann durch Erhöhung der Temperatur beschleunigt werden. Besonders bevorzugt ist die Bildung des Gels bei einer Temperatur kurz unterhalb des Siedepunktes der Synthesemischung. Die Gele werden nach der Herstellung in der Regel bei Temperaturen von 70 bis 150°C, bevorzugt 100 bis 120°C getrocknet.

Die getrockneten Gele können durch Mahlen in den verschiedensten Pulvergrößen hergestellt werden. Relativ feine Pulver erhält man durch Sprühtrocknung der Gele. Eine Aufbringung der Gele vor dem Trocknen auf Träger ist ebenfalls möglich und führt zu Katalysatoren mit schalenförmigem Aufbau.

Durch Kalzinieren der Gele bei Temperaturen von 400 bis 600°C, bevorzugt 500 bis 580°C in oxidierender Atmosphäre kommt man zu den erfindungsgemäßen Katalysatoren, welche entweder direkt oder bevorzugt nach einem Verstrangungsschritt bei der Aminierung eingesetzt werden.

Die Kalzinierung kann auch in mehreren Schritten bei unterschiedlichen Temperaturen und in unterschiedlichen Gasatmosphären erfolgen. Mindestens einer der Kalzinierungsschritte muß jedoch wie oben beschrieben in oxidierend wirkender Atmosphäre bei Temperaturen von 400 bis 600°C erfolgen.

Die durch ein Kalzinieren der getrockneten Gele erhaltenen amorphe Materialien sind folgendermaßen charakterisiert:

Die kalzinierten Gele sind nicht kristallin. Im Röntgenbeugungsdiagramm kann keinerlei Struktur festgestellt werden; die Materialien sind "röntgenamorph".

Die kalzinierten Gele besitzen eine große spezifische Oberfläche. In der Regel ist die nach der sog. BET-Methode [J. Amer. Chem. Soc. **50** (1938) 309 bis 319] bestimmte Oberfläche größer als 180 m²/g, bevorzugt 200 bis 1000 m²/g.

Charakteristisch für einige der erfindungsgemäß hergestellten kalzinierten Gele ist das Vorhandensein von Poren im Größenbereich 2 bis 50 nm, sog. Mesoporen. Das Vorhandensein dieser Mesoporen äußert sich bei Gasadsorptionsmessungen mit Stickstoff bei einer Temperatur von 77 K in einer Adsorptionsisotherme mit Hystereseschleife. Die Adsorptionsisotherme der erfindungsgemäßen kalzinierten Gele hat einen Verlauf, welcher als Typ IV bekannt ist [Klassifizierung der Isothermenformen z.B. in J. Am. Chem. Soc. 62 (1940) 1723]. Eine Hystereseschleife ist auf Kapillarkondensation in den Mesoporen zurückzuführen und fehlt bei nichtmesoporösen Materialien (z.B. Zeolithen).

In der Regel besitzen die kalzinierten Gele auch Mikroporen (Porengröße < 2 nm).

Die Porengrößenverteilung der erfindungsgemäß hergestellten kalzinierten Gele ist üblicherweise deutlich breiter ist als bei Molekularsieben.

Durch die Verstrangung der Gele mit oder ohne Bindemittel, durch eine Sprühtrocknung der Gele oder ein Aufbringen der Gele auf Träger lassen sich weitere Meso- und auch Makroporen in den Katalysator einbringen.

Üblicherweise besitzen die kalzinierten Gele, sofern sie in der H-Form vorliegen, deutlich aciden Charakter. Die Acidität läßt sich beispielsweise nach J. Am. Chem. Soc., 54, 2721 (1932) mit Indikatoren quantifizieren.

Die erfindungsgemäß hergestellten Gele können nach ihrer Trocknung bei 100 bis 160°C, vorzugsweise 100 bis 120°C und Kalzinierung bei 400 bis 600°C, vorzugsweise 500 bis 580°C mit einem Bindemittel zu Strängen oder Tabletten verformt werden. Als Bindemittel eignen sich diverse Aluminiumoxide, bevorzugt Böhmit, Pseudoböhmit, Siliciumdioxid, Titandioxid, Zirkondioxid sowie Tone. Nach der Verformung werden die Extrudate oder Preßlinge getrocknet und kalziniert.

Man erhält auch sehr aktive und selektiv wirkende Katalysatoren, wenn man die Gele direkt nach der Trocknung unter Zugabe eines Bindemittels und/oder Peptisierungsmitttels verformt und erst nach der Verformung einer Kalzinierung unterwirft.

Die Gele könne auch ohne Binder als Stränge oder Tabletten eingesetzt werden, wobei als Peptisierungsmittel z.B. Ethylcellulose, Stearinsäure, Kartoffelstärke, Ameisensäure, Oxalsäure, Essigsäure, Salpetersäure, Ammoniak, Amine, Kieselsäure und Graphit oder deren Gemische verwendet werden können.

Liegt das Gel aufgrund seiner Herstellung nicht in der katalytisch aktiven, aciden H-Form vor, sondern z.B. in der Natrium-Form, dann kann man diese durch Ionenaustausch z.B. mit Ammoniumionen und anschließende Kalzinierung oder durch Behandlung mit Säuren vollkommen oder partiell in die gewünschte H-Form überführen.

Die erhaltenen Gele oder Katalysatoren können nach der Kalzinierung modifiziert werden. Die Modifizierung kann z.B. in einer Dotierung oder einem Ionenaustausch mit einem weiteren Metall bestehen oder in einer thermischen Behandlung in einer Gasatmosphäre, z.B. in Wasserdampf oder in Wasserstoff, bestehen.

Wenn bei den erfindungsgemäßen Katalysatoren eine durch Koksabscheidung bedingte Desaktivierung eintritt, empfiehlt es sich, die Katalysatoren durch Abbrennen der Koksablagerungen mit Luft oder mit einem Luft/Stickstoff-Gemisch bei 400 bis 550°C zu regenerieren, die Katalysatoren erhalten dadurch ihre Anfangsaktivität zurück.

Die hier beschriebenen Katalysatoren können wahlweise als Stränge oder als Tabletten mit 1 bis 5 mm Durchmesser oder als Splitt oder als Wirbelkontakt eingesetzt werden.

Die Substituenten R¹ und R² sowie der Index n in den Verbindungen der allgemeinen Formeln I, II und III haben die folgenden Bedeutungen:
R¹, R², R³, R⁴, R⁵ und R⁶
- unabhängig voneinander
- Wasserstoff,
- C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl,sec.-Butyl und tert.-Butyl,
- C₁- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- C₄- bis C₂₀-Alkyl-cycloalkyl, bevorzugt C₆ bis C₂₀-Alkylcycloalkyl wie 2-Methyl-cyclopentyl, 3-Methylcyclohexyl und 4-Methylcyclohexyl,
- C₄- bis C₂₀-Cycloalkyl-alkyl, bevorzugt C₆- bis C₂₀-Cycloalkylalkyl wie Cyclopentyl-methyl, Cyclohexyl-methyl und Cyclohexyl-ethyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- einen heterocyclischen Rest wie einen aromatischen und einen nicht-aromatischen Heterocyclus mit ein bis drei Heteroatomen wie Stickstoff, Sauerstoff und/oder Schwefel, bevorzugt Stickstoff und Sauerstoff.

Als Olefine II seien vorzugsweise genannt:

Ethen, n-Propen, iso-Propen, But-1-en, iso-Buten, But-2-en, Diisobuten, Cyclopenten, Cyclohexen und Polyisobuten.

Als Verbindung III eignet sich bevorzugt Ammoniak.

Als aliphatische Amine III sind vorzugsweise genannt: Methylamin, Ethylamin, N-Propylamin, iso-Propylamin, N-Butylamin, iso-Butylamin, sec.-Butylamin, N-2-Methyl-2-propylamin, N-3-Methyl-1-butylamin, n-Hexylamin, n-Octylamin, 2-Ethylhexylamin, n-Tridecylamin, Dimethylamin, Diethylamin, Di-n-propylamin, Di-iso-propylamin, Di-n-butylamin, Di-iso-butylamin, Di-sec.-butylamin, N-Methyl-N-n-butylamin, N-Ethyl-N-n-butylamin und Anilin.

Als cyclische Amine III seien vorzugsweise genannt:

Pyrrolidin, Morpholin und Piperidin, Dihydropyrrol und Tetrahydropyrrol.

Die Herstellung derartiger Ausgangsstoffe der Formeln II und III ist hinlänglich aus Standardwerken (Beilstein, Gmelin) bekannt.

Die Amine I sind in der Regel wertvolle Bausteine in der organischen Synthese. Besonders interessant sind solche Verbindungen als Vorprodukte für Pharmazeutika und Wirkstoffe in Herbiziden, Fungiziden sowie Insektiziden und als Katalysatoren für die organische Synthese bzw. bei Polymerisationen.

### Beispiele

### Herstellung der Katalysatoren

### Katalysator A

In 68,5 g einer 13,35 %igen Tetrapropylammoniumhydroxid-Lösung wurden 0,62 g Borsäure gelöst. Die Lösung wurde auf 60°C erwärmt und 104,1 g Tetraethyl-ortho-silikat wurden unter Rühren zugegeben. Nach 15 Minuten bei 60°C bildete sich ein homogenes Gel, welches bei 110°C getrocknet wird.

Eine kleine Probe des getrockneten Gels wird bei 550°C in Luft kalziniert und charakterisiert: Das kalzinierte Material ist röntgenamorph und besitzt eine BET-Oberfläche von 670 m²/g. Das Material zeigt eine Adsorptionsisotherme mit Hystereseschleife. Aus dieser Isotherme kann eine Porengrößenverteilung im Mesoporenbereich mit einem deutlichem Maximum bei 30 bis 40 Å berechnet werden. Die chemische Zusammensetzung des Materials ist ungefähr SiO₂ · 0,01 B₂O₃.

Zum Einsatz bei der Aminierungsreaktion wird das Boroxid-haltige Kieselgel wie folgt zu einem Formkörper verstrangt:

40 g des getrockneten Gel werden in einer Kugelmühle auf eine Größe von 10 bis 50 µm gemahlen. In einem Kneter werden zu dem zerkleinerten Gel 20 g Pseudoböhmit gegeben und die Masse mit Wasser und etwas Ameisensäure zu einer viskosen Paste verarbeitet.

Die Paste wird verstrangt, bei 110°C 5 Stunden lang getrocknet und 8 Stunden bei 550°C in Luft kalziniert.

### Katalysator B

In 68,5 g einer 15 %igen Ammoniak-Lösung wurden 6 g Aluminiumtriisopropanolat gelöst. Die Lösung wurde auf 60°C erwärmt und 104,1 g Tetraethyl-ortho-silikat wurden unter Rühren zugegeben.

Nach 20 Minuten bei 60°C bildete sich ein homogenes Gel, welches bei 110°C getrocknet wird. Eine kleine Probe des getrockneten Gels wird bei 550°C in Luft kalziniert und charakterisiert: Das kalzinierte Material ist röntgenamorph, besitzt eine BET-Oberfläche von 580 m²/g und besitzt eine Adsorptionsisotherme mit Hystereseschleife. Die chemische Zusammensetzung des Materials ist ungefähr SiO₂ · 0,03 Al₂O₃.

Zum Einsatz bei der Aminierungsreaktion wird das Aluminiumoxidhaltige Kieselgel wie bei Katalysator A beschrieben zu einem Formkörper verstrangt.

### Katalysator C

10 g pyrogene Kieselsäure (Aerosil^{R}) wurden in 235 g Tetrapropylammoniumhydroxid (FLUKA, 20 %ig) aufgelöst. Zu dieser Lösung werden 0,5 g Aluminiumhydroxid gegeben und heftig gerührt.

Nach langsamer Zugabe von insgesamt 200 ml einer 1-molaren Salzsäure-Lösung gelierte die Lösung.

Das Gel wurde bei Raumtemperatur 24 Stunden gealtert und anschließend bei 110°C getrocknet.

Ungefähre Zusammensetzung des Materials: SiO₂ · 0,02 Al₂O₃.

Zum Einsatz bei der Aminierungsreaktion wird das Aluminiumoxidhaltige Kieselgel wie bei Katalysator A beschrieben zu einem Formkörper verstrangt.

### Katalysator D

46 g Aluminiumtriisopropanolat, 23 g Phosphorsäure (85 %ig), 12 g Kieselsol (15 %ig) und 28,7 g Tri-n-propylamin (TPA) wurden unter Rühren zu 44 g Wasser gegeben. Die Mischung wurde homogenisiert und bei 50°C 15 Minuten behandelt, wobei sie gelierte. Die ungefähre chemische Zusammensetzung des kalzinierten Gels betrug: Al₂O₃ · 0,9 P₂O₅ · 0,3 SiO₂. Zum Einsatz bei der Aminierungsreaktion wird das Siliciumoxid-haltige Aluminiumphosphat-Gel wie bei Katalysator A beschrieben zu einem Formkörper verstrangt.

### Katalysator E

88,2 g Natriumaluminat, 45,6 g Natriumhydroxid und 384 g Wasser wurden auf 60 bis 80°C erhitzt und mit einem Intensivrührer gerührt. Zu dieser Lösung wird schnell eine 100°C heiße Lösung aus 360 g Natrium-Wasserglas, 638 g Wasser und 45,6 g Natronlauge zugegeben. Die Mischung wurde 5 Minuten homogenisiert, wobei sie gelierte. Anschließend wird das Gel 16 Stunden bei Raumtemperatur gealtert. Das Gel wird 16 h bei 550°C kalziniert und zum Ionenaustausch mit einer 1-molaren Ammoniumnitrat-Lösung 2 Stunden lang gerührt. Dieser Prozeß wird dreimal wiederholt Das Material wird bei 550°C kalziniert und die Ammoniumnitrat-Behandlung wiederholt.

Die ungefähre chemische Zusammensetzung des kalzinierten Gels betrug: SiO₂ · 0,3 Al₂O₃.

Zum Einsatz bei der Aminierungsreaktion wird das ionenausgetauschte Aluminiumoxid-haltige Kieselgel wie bei Katalysator A beschrieben zu einem Formkörper verstrangt.

### Katalysator F

In 960 g einer 50 %igen Hexamethylendiamin-Lösung werden 74 g pyrogene Kieselsäure suspendiert. Zu dieser Suspension wurde eine Lösung von 14 g Borsäure in 80 g 50 %iger Hexamethylendiamin-Lösung gegeben. Die Lösung wurde unter Rühren auf 60°C erwärmt, wobei sie nach 60 Minuten gelierte. Das Gel wurde 16 Stunden bei Raumtemperatur gealtert und bei 110°C getrocknet.

Die ungefähre chemische Zusammensetzung des kalzinierten Gels betrug: SiO₂ · 0,03 B₂O₃.

Zum Einsatz bei der Aminierungsreaktion wird das Bor-haltige SiO₂-Gel wie bei Katalysator A beschrieben zu einem Formkörper verstrangt.

### Katalysator G

Katalysator G wird erhalten, indem an 50 g Katalysator C eine Behandlung mit 140 ml 0,1n HF unter Rückfluß für 1h vorgenommen wird. Nach der Filtration und Auswaschen mit Wasser wird bei 110°C/16h getrocknet und bei 500°C/5h kalziniert.

### Katalysator H

Katalysator H wird erhalten, indem man 50 g Katalysator C einer Behandlung mit 2 Gew.-% H₃BO₃ unterzieht. Nach der Filtration und Auswaschen mit Wasser wird bei 110°C/16h getrocknet und bei 500°C/5h kalziniert.

### Katalysator I

Katalysator I wird erhalten, indem man 50 g Katalysator F einer Behandlung mit 2 Gew.-% H₃BO₃ unterzieht. Nach der Filtration und Auswaschen mit Wasser wird bei 110°C/16h getrocknet und bei 500°C/5h kalziniert.

### Katalysator J

Katalysator J wird erhalten, indem man 50 g Katalysator B einer Behandlung mit 2 Gew.-% H₃PO₄ unterzieht. Nach der Filtration und Auswaschen mit Wasser wird bei 110°C/16h getrocknet und bei 500°C/5h kalziniert.

### Katalysator K

240 g Tetramethoxysilan (Si(OCH₃)₄) werden mit 21,8 g Borsäuretrimethylester (B(OCH₃)₃) und 60 g Sokalan CP 10 S® (50 % Polyacrylsäure in Wasser) gemischt. Nach 30 min Rühren werden 380 g Wasser und 13 g 0,1 N Flußsäure dazugegeben. Nach weiteren 15 min wird das Rühren beendet und der Ansatz ausgelieren gelassen. Das Gel wurde 2 h bei 120°C getrocknet, anschließend zermahlen und 12 h bei 350°C calciniert.

Verstrangung analog zu A, Calcinierung 5 h 500°C, BET 326 m²g⁻¹.

### Katalysator L

36 g Aluminiumtriisopropylat werden in 360 g Isopropanol bei 70°C gelöst und die Lösung dann auf Raumtemperatur abgekühlt. 180 g Tetramethoxysilan, 180 g Wasser und 7,7 ml 0,1 N Flußsäure werden in dieser Reihenfolge langsam hinzugegeben. Nach einiger Zeit Rühren bei Raumtemperatur wird der Ansatz auf 50°C erwärmt und ausgeliert. Das Gel wurde 2 h bei 120°C getrocknet, anschließend zermahlen und 12 h bei 350°C calciniert.

Verstrangung analog zu A, Calcinierung 5 h 500°C, BET 228 m²g⁻¹.

### Katalysator Y (Vergleichskatalysator)

handelsübliches amorphes Alumosilikat (BET = 180 m²/g)

### Katalysator Z (Vergleichskatalysator)

Aluminiumzeolith vom Pentasil-Typ (SiO₂/Al₂O₃ = 40)

### Beispiele 1 bis 32

Die Versuche werden in einer kontinuierlichen Hochdruckapparatur durchgeführt. Das Reaktorvolumen ist 100 ml, die Beheizung erfolgt mittels Alublockheizung. Eine dreifache Innentemperaturmessung wird durchgeführt. Die Druckhaltung erfolgt bei 300 bar. Der Zulauf des Eduktgemisches (Isobuten und Ammoniak) erfolgt von oben. Die Katalysatorfüllmenge beträgt etwa 60 ml. Das restliche Reaktorvolumen wird mit Inertmaterial (Glaskugeln, Porzellanringe, etc.) befüllt. Die Austräge werden gaschromatographisch getrennt nach Flüssig- und Gasphase untersucht. Das Ammoniak zu Isobutenverhältnis beträgt 1,5.

**Tabelle 1**

| Beispiel Nr. | Katalysator | Temperatur [°C] | WHSV (g-Edukt/h/g-Katalysator) | Ausbeute [%] |
|---|---|---|---|---|
| 1 | A | 270 | 0,7 | 2,8 |
| 2 | A | 270 | 1,5 | 1,4 |
| 3 | A | 270 | 3,0 | 0,8 |
| 4 | A | 300 | 0,7 | 11,4 |
| 5 | A | 300 | 1,5 | 8,8 |
| 6 | A | 300 | 3,0 | 5,8 |
| 7 | B | 270 | 0,2 | 17,0 |
| 8 | B | 270 | 0,5 | 11,7 |
| 9 | B | 270 | 1,0 | 1,5 |
| 10 | B | 300 | 0,7 | 15,2 |
| 11 | B | 300 | 1,5 | 14,8 |
| 12 | B | 300 | 3,0 | 13,4 |
| 14 | C | 270 | 0,7 | 12,2 |
| 17 | C | 300 | 1,5 | 12,6 |
| 19 | D | 270 | 1,5 | 0,5 |
| 20 | D | 300 | 1,5 | 2,1 |
| 21 | E | 270 | 1,5 | 2,6 |
| 22 | E | 300 | 1,5 | 2,2 |
| 23 | F | 270 | 1,5 | 5,4 |
| 25 | G | 270 | 0,7 | 14,1 |
| 26 | H | 270 | 0,7 | 12,4 |
| 27 | I | 270 | 0,7 | 9,4 |
| 28 | J | 270 | 0,7 | 14,3 |
| 29 | Y | 270 | 1,5 | 0,3 |
| 30 | K | 300 | 0,7 | 13 |
| 31 | K | 300 | 1,5 | 10 |
| 32 | K | 300 | 3 | 7 |
| 33 | L | 300 | 0,7 | 12 |
| 34 | L | 300 | 1,5 | 10 |
| 35 | L | 300 | 3 | 7 |
| 36 | Y | 300 | 1,5 | 2,7 |
| 37 | Z | 270 | 1,5 | 16,7 |
| 38 | Z | 300 | 1,5 | 12,9 |

### Beispiele 39

Die Versuche werden in einer kontinuierlichen Hochdruckapparatur durchgeführt. Das Reaktorvolumen ist 100 ml, die Beheizung erfolgt mittels Alublockheizung. Eine dreifache Innentemperaturmessung wird durchgeführt. Die Druckhaltung erfolgt bei 300 bar. Der Zulauf des Eduktgemisches (Cyclopenten und Ammoniak) erfolgt von oben. Die Katalysatorfüllmenge beträgt etwa 60 ml. Das restliche Reaktorvolumen wird mit Inertmaterial (Glaskugeln, Porzellanringe, etc.) befüllt. Die Austräge werden gaschromatographisch getrennt nach Flüssig- und Gasphase untersucht. Das Ammoniak zu Cyclopentenverhältnis beträgt 1,5.

**Tabelle 2**

| Beispiel Nr. | Katalysator | Temperatur [°C] | WHSV (g-Edukt/h/g-Katalysator | Ausbeute [%] |
|---|---|---|---|---|
| 40 | B | 270 | 0,7 | 5,6 |

## Patentansprüche

1. Verfahren zur Herstellung von Aminen der allgemeinen Formel I 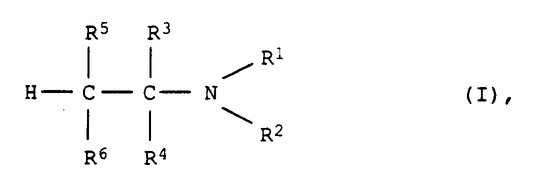 in der
R¹,R²,R³,R⁴,R⁵,R⁶ Wasserstoff, C₁- bis C₂₀-Alkyl, C₃- bis C₂₀-Cycloalkyl, C₄- bis C₂₀-Alkyl-cycloalkyl, C₄- bis C₂₀-Cycloalkyl-alkyl, Aryl, C₇-bis C₂₀-Alkylaryl, C₇- bis C₂₀-Aralkyl und heterocyclische Reste
bedeuten, dadurch gekennzeichnet, daß man Olefine der allgemeinen Formel II 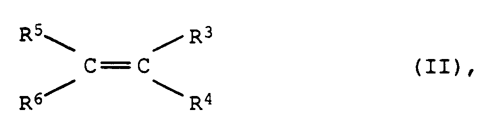 in der R³, R⁴, R⁵ und R⁶ oben genannten Bedeutung haben, mit Ammoniak, primären oder sekundären Aminen der allgemeinen Formel III 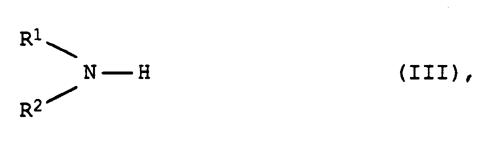 in der R¹ und R² die oben genannten Bedeutungen hat, bei Temperaturen von 200 bis 400°C und Drücken von 1 bis 700 bar in Gegenwart von mesoporösen und gegebenenfalls mikroporösen, röntgenamorphen Katalysatoren der Zusammensetzung
a MO₂ * b Q₂O₃ * c P₂O₅ (IV),
in der Q für Aluminium, Bor, Chrom, Eisen oder Gallium und M für Silicium, Titan oder Germanium steht, und das Molverhältnis a zu b 0,5:1 bis 1000:1 und das von c zu b 0 bis 2:1 beträgt, mit einer spezifische BET-Oberfläche von 200 bis 1000 m²/g, umsetzt.

2. Verfahren zur Herstellung von Aminen nach Anspruch 1, dadurch gekennzeichnet, daß man Katalysatoren IV einsetzt, die durch Hydrolyse einer Wasser, gegebenenfalls einem weiteren Lösungsmittel, einer oder mehreren löslichen, hydrolysierbaren Q-Verbindungen und einer oder mehreren löslichen, hydrolysierbaren M-Verbindungen enthaltenden Synthesemischung, hergestellt wird.

## Claims

1. A process for preparing amines of the general formula I 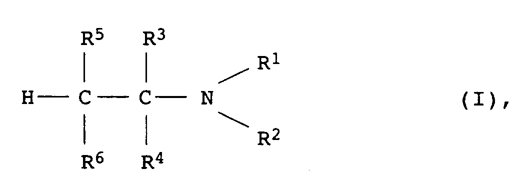 where
R¹,R²,R³,R⁴,R⁵,R⁶ are hydrogen, C₁- to C₂₀-alkyl, C₃- to C₂₀-cycloalkyl, C₄- to C₂₀-alkyl-cycloalkyl, C₄- to C₂₀-cycloalkyl-alkyl, aryl, C₇- to C₂₀-alkylaryl, C₇- to C₂₀-aralkyl and heterocyclic radicals,
which comprises reacting olefins of the general formula II 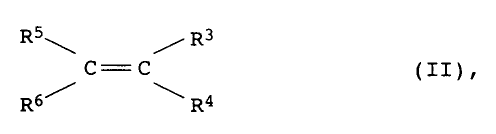 where R³, R⁴, R⁵ and R⁶ are as defined above, with ammonia or primary or secondary amines of the general formula III 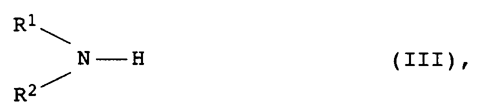 where R¹ and R² are as defined above, at from 200 to 400°C and pressures of from 1 to 700 bar in the presence of mesoporous and, if desired, microporous, X-ray-amorphous catalysts having the composition
a MO₂ * b Q₂O₃ * c P₂O₅ (IV),
where Q is aluminum, boron, chromium, iron or gallium and M is silicon, titanium or germanium, and the molar ratio of a to b is from 0.5:1 to 1000:1 and that of c to b is from 0 to 2:1, and having a specific BET surface area of from 200 to 1000 m²/g.

2. A process for preparing amines as claimed in claim 1, wherein the catalysts IV used are prepared by hydrolysis of a synthesis mixture comprising water, if desired a further solvent, one or more soluble, hydrolyzable Q compounds and one or more soluble, hydrolyzable M compounds.

## Revendications

1. Procédé pour la préparation d'amines de formule générale I 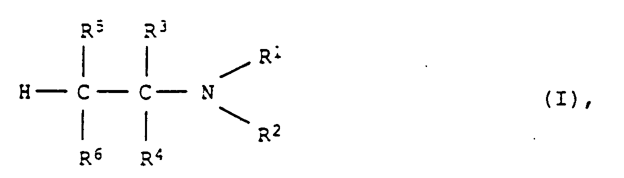 dans laquelle
R¹, R², R³, R⁴, R⁵, R⁶ représentent chacun l'hydrogène, un groupe alkyle en C1-C20, cycloalkyle en C3-C20, (alkylcycloalkyle en C4-C20, cycloalkylalkyle en C4-C20, aryle, alkylaryle en C7-C20, aralkyle en C7-C20 ou un groupe hétérocyclique,
caractérisé par le fait que l'on fait réagir des oléfines de formule générale II 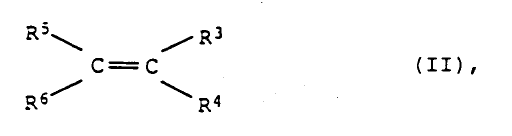 dans laquelle R³, R⁴, R⁵, R⁶ ont les significations indiquées ci-dessus, avec l'ammoniac, des amines primaires ou secondaires de formule générale III 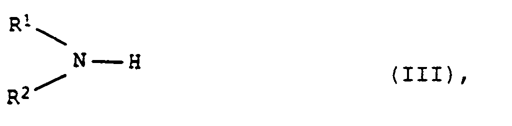 dans laquelle R¹ et R² ont les significations indiquées ci-dessus, à des températures de 200 à 400° C et des pressions de 1 à 700 bar en présence de catalyseurs mésoporeux et le cas échéant microporeux, amorphes aux rayons X, de composition
a MO₂ * b Q₂O₃ * c P₂O₅ (IV),
dans laquelle Q représente l'aluminium, le bore, le chrome, le fer ou le gallium et M représente le silicium, le titane ou le germanium, et le rapport molaire a/b va de 0,5 : 1 à 1000 : 1, le rapport molaire c/b va de 0 à 2 : 1, et la surface spécifique BET est de 200 à 1000 m²/g.

2. Procédé pour la préparation d'amines selon revendication 1, caractérisé par le fait que l'on utilise des catalyseurs IV qui ont été préparés par hydrolyse d'un mélange de synthèse contenant de l'eau, le cas échéant un autre solvant, un ou plusieurs dérivés solubles hydrolysables de Q et un ou plusieurs dérivés solubles hydrolysables de M.
